# EUROPÄISCHE PATENTSCHRIFT

(11) **EP 1 483 235 B1**
(45) Veröffentlichungstag und Bekanntmachung des Hinweises auf die Patenterteilung: **09.06.2010**
(21) Anmeldenummer: 03709737.5
(22) Anmeldetag: 28.02.2003
(51) Int. Cl.: C07C 263/04

(54) **VERFAHREN ZUR HERSTELLUNG VON ISOCYANATEN**
PROCESS FOR THE PREPARATION OF ISOCYANATES
PROCEDE DE PRODUCTION D'ISOCYANATES

(30) Priorität: 01.03.2002 DE 10209095
(43) Veröffentlichungstag der Anmeldung: 08.12.2004
(73) Patentinhaber: BASF SE, 67056 Ludwigshafen (DE)
(72) Erfinder: STRÖFER, Eckhard, 68163 Mannheim (DE); OCHSE, Michael, 67098 Bad Dürkheim (DE); KRASE, Volker, 01979 Lauchhammer (DE); SCHMIDT, Andreas, 01987 Schwarzheide (DE); KLÖTZER, Matthias, 01945 Kroppen (DE); MURRAR, Imbridt, 01968 Senftenberg (DE); FRANZKE, Gisbert, 01987 Schwarzheide (DE)
(86) Internationale Anmeldenummer: PCT/EP2003/002131
(87) Internationale Veröffentlichungsnummer: WO 2003/074477

(56) Entgegenhaltungen:
- EP-A- 0 524 554
- EP-A- 0 568 782

## Beschreibung

Die Erfindung betrifft ein Verfahren zur Herstellung von Isocyanaten durch thermische Spaltung von Carbamidsäureestern.

Es ist bekannt, Isocyanate nach einem Verfahren herzustellen, das ohne den Einsatz von Phosgen auskommt. Dabei wird ein Amin mit einem Alkohol und Harnstoff zum entsprechenden Carbamidsäureester (Urethan) umgesetzt. Anschließend wird der Carbamidsäureester thermisch zum Isocyanat gespalten, wobei der Alkohol wieder freigesetzt wird.

Die Spaltung der Carbamidsäureester kann in der Gasphase oder in flüssiger Phase geschehen. Die Spaltung der Carbamidsäureester nach Verdampfung in der Gasphase, beschrieben beispielsweise in US 3,734,941, bringt den Nachteil der Verstopfungsgefahr in dem vorgeschalteten Verdampfer mit sich. Die Spaltung in flüssiger Phase macht es erforderlich, eines der beiden Produkte der Spaltreaktion aus dem Reaktionsgemisch zu entfernen, um eine Rückreaktion zu verhindern. Üblicherweise werden beide Spaltprodukte gasförmig entnommen und anschließend getrennt.

Für die Herstellung mehrfach funktioneller Isocyanate, bei der die Gefahr der Polymerisation und Rückstandsbildung verstärkt besteht, wird in DE-A 24 21 503 die Verdünnung der Carbamidsäureester mit inerten Lösungsmitteln vorgeschlagen. Die Mitverwendung von inerten Lösungsmitteln bedeutet einen erhöhten Aufwand. In der EP-A 92 738 wird die lösungsmittelfreie Spaltung vorgeschlagen. Als geeignete Spaltreaktoren werden Dünnschichtverdampfer und Fallfilmverdampfer genannt.

EP-A 0 524 554 offenbart ein Verfahren zur lösungsmittelfreien Spaltung von Carbamidsäureestern. Dabei wird Hexamethylendi-n-butylurethan kontinuierlich in einem Heizkerzenreaktor bei 240°C und 30 mbar gespalten. Die Spaltgase werden in einer Rektifizierkolonne, die sich auf dem Reaktor befindet und einen Rücklauf und einen Seitenabzug aufweist, aufgetrennt.

Die in EP 0 568 782 beschriebene Zwischenkühlung mit Wärmerückgewinnung im unteren Drittel einer kombinierten Spalt- und Rektifizierkolonne mittels eines Rohrbündelwärmetauschers besitzt den Nachteil von Anbackungen und/oder Verstopfung des Apparates. Durch die Aufgabe des zu spaltenden Urethanes oberhalb des Wärmetauschers wird diesen Nachteil noch verstärkt.

Aufgabe der Erfindung ist es, ein verbessertes Verfahren zur lösungsmittelfreien Spaltung von Carbamidsäureestern bereitzustellen.

Gelöst wurde die Aufgabe durch ein Verfahren zur Herstellung von Isocyanaten aus Carbamidsäureestern, bei dem man
(i) ein Gemisch enthaltend einen einfach oder mehrfach funktionellen Carbamidsäureester kontinuierlich in einen Spaltreaktor oder eine Kaskade aus zwei oder mehr Spaltreaktoren einleitet,
(ii) den Carbamidsäureester einer thermischen Spaltung zu Isocyanat und Alkohol unterwirft,
(iii) das dem oder den Spaltreaktoren entweichende Spaltgas, enthaltend das Isocyanat, den Alkohol, gegebenenfalls ungespaltenen und/oder teilweise gespaltenen Carbamidsäureester sowie Nebenkomponenten, zumindest teilweise mit einem flüssigen Kühl- und Kondensationsmittel in Kontakt bringt, abkühlt und zumindest teilweise kondensiert,
(iv) aus dem abgekühlten Spaltgas und dem Kondensat in einer Rektifizierkolonne das Isocyanat und den Alkohol gewinnt,
wobei ein Teilstrom des gewonnenen Alkohols als flüssiges Kühl- und Kondensationsmittel zur Abkühlung und Kondensation des Spaltgases eingesetzt wird.

Überraschender Weise wurde gefunden, dass sich die Isocyanat-Ausbeute der Carbamidsäureesterspaltung und anschließenden Auftrennung der Spaltprodukte in einer Rektifizierkölonne erheblich verbessern lässt, wenn die Spaltgase vor Durchführung der Rektifikation mit einem flüssigen Kühl- und Kondensationsmittels abgekühlt und dabei zumindest teilweise kondensiert werden. Das Kühl- und Kondensationsmittel ist zweckmäßiger Weise der in der Spaltungsreaktion freigesetzte Alkohol, der in der Rektifizierkolonne gewonnen wird.

Die Spaltgase enthalten neben dem Isocyanat und dem Alkohol üblicherweise auch ungespaltenes und - bei Spaltung von mehrfach funktionellen Carbamidsäureestern - auch teilweise gespaltene Carbamidsäureester, beispielsweise bei der Spaltung von Diurethanen das Monourethan. Ferner können die Spaltgase Nebenbestandteile wie Carbonate, Carbamate und Kohlendioxid enthalten.

Carbamidsäureester, die nach dem erfindungsgemäßen Verfahren gespalten werden, sind monofunktionelle oder polyfunktionelle Carbamidsäureester, bevorzugt bifunktionelle Carbamidsäureester. Beispiele für Isocyanate, die nach dem erfindungsgemäßen Verfahren hergestellt werden, sind 1,4-Diisocyanatobutan, 1,6-Diisocyanatohexan (HDI), 2-Methyl-1,5-diisocyanatopentan, 1,5-Diisocyanato-2,2-dimethylpentan, 2,4,4-Trimethyl-1,6-diisocyanatohexan, 1,10-Diisocyanatodecan, 1,3- und 1,4-Diisocyanatocyclohexan, 1,3- und 1,4-Bis-(isocyanatomethyl)-cyclohexan, 1-Isocyanato-3,3,5-trimethyl-5-isocyanatomethylcyclohexan (Isophorondiisocyanat, IPDI), 4,4'-Diisocyanatodicyclohexylmethan, 1-Isocyanato-1-methyl-4(3)isocyanatomethylcyclohexan (IMCI), Bis-(isocyanatomethyl)-norboman, 2,4- und 2,6-Diisocyanatotoluol (TDI), 2,4'- und 4,4'-Diisocyanatodiphenylmethan und höhere Homologe, 1,5-Diisocyanatonaphthalin, Dipropylenglycoldiisocyanat, Triisocyanate und/oder höherfunktionelle Isocyanate wie z.B. 4-Isocyanatomethyl-1,8-octandiisocyanat (Nonantriisocyanat), 1,6,11-Undecantriisocyanat oder beliebige Gemische solcher Isocyanatverbindungen. Beispiele für Alkohole, die in den erfindungsgemäß zu spaltenden Carbamidsäureestern enthalten sind und als Kühl- und Kondensationsmittel fungieren, sind Methanol, Ethanol, n-Propanol und n-Butanol. Besonders bevorzugter Carbamidsäureester ist Hexamethylendi-n-butylurethan (HDU).

Der Kühl- und Kondensationsschritt (iii) kann in einem Separator erfolgen, der der Rektifizierkolonne vorgeschaltet ist. Dabei wird der flüssige Alkohol als Kühl- und Kondensationsmittel mittels eines geeigneten Verteilers, beispielsweise einer Sprühdüse, in den heißen Gasstrom, der den Spaltreaktor verlässt, eingespeist. Die so abgekühlten Gase können mittels eines Wärmetauschers, beispielsweise eines Rohrbündelwärmetauschers, weiter abgekühlt werden.

Vorzugsweise wird der Kondensationsschritt (iii) in der Rektifizierkolonne selbst durchführt. Dazu wird das Spaltgas am Kolonnensumpf in die Rektifizierkolonne eingespeist und der Teilstrom des gewonnenen Alkohols, der als Kühl- und Kondensationsmittel fungiert, im unteren Kolonnenabschnitt, vorzugsweise auf Höhe des 1. bis 4. theoretischen Bodens, insbesondere in der Höhe des 1. theoretischen Bodens, in die Rektifizierkolonne eingespeist und dem aufsteigenden Spaltgas entgegengeführt.

Geeignete Rektifizierkolonnen sind übliche Boden-, Packungs- oder Füllkörperkolonnen und weisen üblicherweise von 8 bis 25 theoretische Böden auf.

Die Rektifizierkolonne weist einen Rücklauf am Kolonnenkopf, einen Seitenabzug zur Entnahme des höher siedenden Spaltproduktes (Isocyanat oder Alkohol) und einen Kopfabzug zur Entnahme des niedriger siedenden Spaltproduktes auf. Üblicherweise ist der Alkohol das niedriger siedende Spaltprodukt und wird am Kolonnenkopf als Kopfabzugsstrom erhalten. Das Isocyanat wird entsprechend als Seitenabzugsstrom gewonnen. Es ist jedoch auch der umgekehrte Fall möglich.

Das Rücklaufverhältnis beträgt üblicherweise von 1 bis 4. Von dem als Kopf- bzw. Seitenabzugsstrom, vorzugsweise als Kopfabzugsstrom, entnommenen Alkohol wird üblicherweise ein Teilstrom von 5 bis 40 % als Kühl- und Kondensationsmittel entnommen und in den unteren Teil der Kolonnen eingespeist.

Am Sumpf der Kolonne werden üblicherweise ungespaltene und gegebenenfalls teilgespaltene Carbamidsäureester erhalten. Diese werden vorzugsweise in den Spaltreaktor zurückgeführt.

Geeignete Spaltreaktoren sind solche, die Einbauten zum Einbringen der Reaktionswärme enthalten. Beispiele sind Robertverdampfer, Herbertverdampfer, Heizkerzenverdampfer, Caddletype-Verdampfer, Rohrbündelreaktoren, Kessel mit innenliegender Heizschlange und ähnliche Reaktoren, die sich durch einen hohen Wärmeeintrag in ein kleines Volumen auszeichnen. Das Reaktionsmedium liegt darin in einem siedeähnlichen Zustand vor, bei dem die aus der Spaltreaktion resultierenden Gase und Dämpfe des Eduktes gebildet werden und in der Flüssigkeit aufsteigen. Vorzugsweise werden die Reaktoren, wie in EP-A 0 524 554 beschrieben, so betrieben, dass die zweiphasige Mischung aus siedender Flüssigkeit und Dampfphase aus dem verdampften Edukt und den Spaltgasen einen volumetrischen Gasanteil von über 50 % enthält. Die Verweilzeit der siedenden Flüssigkeit in dem Reaktor beträgt 1 bis 60 min, bevorzugt 5 bis 30 min. Die Reaktion wird ohne jeden Zusatz von Lösungs- oder Verdünnungsmittel durchgeführt.

Die Erfindung wird durch die nachstehenden Beispiele näher erläutert.

### Beispiele

### Vergleichsbeispiel 1

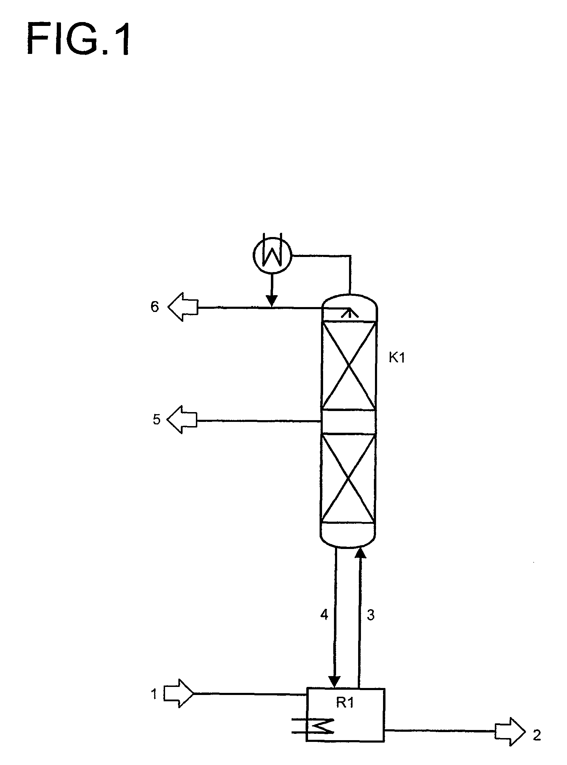

Die Spaltreaktion und die Aufarbeitung der Spaltgase wird in der in Figur 1 dargestellten Vorrichtung durchgeführt. In den Spaltreaktor R1 werden 4 kg/h eines Gemischs aus 80 Gew.-% 2,4-Toluylendi-n-butylurethan und 20 Gew.-% 2,6-Toluylendi-n-butylurethan als Strom 1 eingespeist. Die Reaktortemperatur beträgt ca. 240 °C, der Reaktordruck ca. 100 mbar und die mittlere Verweilzeit des Reaktionsgemischs in dem Spaltreaktor ca. 10 min. Der thermische Leistungseintrag beträgt ca. 3,4 kW. Die Spaltgase werden in der Rektifizierkolonne K1, die 10 theoretische Trennstufen besitzt, aufgetrennt. Der Spaltgasstrom 3 wird in den Sumpf der Kolonne eingespeist. Auf Höhe der 5. theoretischen Stufe wird ein Seitenabzugsstrom 5 von 1,3 kg/h Toluylendiisocyanat (TDI) mit einer Reinheit von 93 Gew.-% abgezogen. Als Kopfabzugsstrom 6 werden 1,6 kg/h n-Butanol mit einer Reinheit von 98 Gew.-% entnommen. Die Temperatur im Kopfkondensator beträgt ca. 36 °C, der Druck beträgt ca. 20 mbar. Der Sumpfabzugstrom 4 von ca. 14 kg/h enthält ca. 70 Gew.-% TDI und 20 Gew.-% halbgespaltenes Urethan und wird in den Reaktor R1 zurückgeleitet. Der Reaktoraustragsstrom 2 von ca. 1,1 kg/h enthält ca. 80 Gew.-% niedermolekulare und hochmolekulare Allophanate.

Die Ausbeute an Toluylendiisocyanat, bezogen auf eingesetztes Toluylendi-n-butylurethan, beträgt ca. 60,8 %.

### Beispiel 1

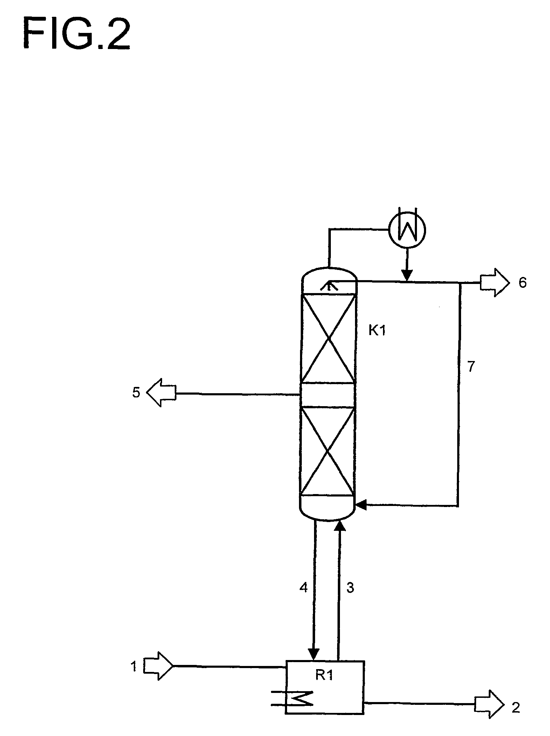

Die Spaltreaktion und Aufarbeitung der Spaltprodukte wird in der in Figur 2 dargestellten Vorrichtung durchgeführt. Diese gleicht der Vorrichtung gemäß Figur 1 (Vergleichsbeispiel), jedoch wird auf Höhe der 1. theoretischen Stufe ein Strom 7 von 0,8 kg/h flüssigem n-Butanol, der dem Kopfabzugsstrom entnommen wird, als Kühl- und Kondensationsmittel eingespeist. Bei sonst gleichen Betriebsbedingungen (Reaktorzulauf 1 = 4 kg/h, Reaktortemperatur = 240 °C, Leistungseintrag 3,9 kW, Kopfabzugsstrom 6 = 1,6 kg/h, Kolonnendruck 20 mbar) konnte der Seitenabzugsstrom 5 von 1,3 kg auf 1,5 kg gesteigert werden. Der Reaktoraustragsstrom 2 reduzierte sich dadurch von ca. 1,1 kg/h auf ca. 0,9 kg/h.

Die Ausbeute an Toluylendiisocyanat verbesserte sich dadurch von 60,8 auf 66,2 %.

### Vergleichsbeispiel 2

Die Spaltreaktion und die Aufarbeitung der Spaltgase wird in der in Figur 1 dargestellten Vorrichtung durchgeführt. In den Spaltreaktor R1 werden 4 kg/h eines Gemisches aus 89 Gew.-% Hexamethylendi-n-butylurethan als Strom 1 eingespeist. Die Reaktortemperatur beträgt 238°C, der Reaktordruck ca. 35 mbar und die mittlere Verweilzeit des Reaktionsgemisches in dem Spaltreaktor ca. 25 min. Der thermische Leistungseintrag beträgt ca. 1,4 kW. Die Spaltgase werden in der Rektifizierkolonne K1, die 25 theoretische Trennstufen besitzt, aufgetrennt. Der Spaltgasstrom 3 wird in den Sumpf der Kolonne eingespeist. Auf der Höhe der 14. theoretischen Stufe wird ein Seitenabzugsstrom 5 von ca. 1,3 kg/h Hexamethylendiisocyanat mit einer Reinheit von 97,5 Gew.-% abgezogen. Als Kopfabzugsstrom 6 werden ca. 1,4 kg/h n-Butanol mit einer Reinheit von 97 Gew.-% entnommen. Die Temperatur im Kopfkondensator beträgt ca. 28°C und der Druck etwa 20 mbar. Der Abzugsstrom 4 aus der Kolonne von 3,7 kg/h enthält ca. 60 Gew.-% halbgespaltenes Urethan, 15 Gew.-% Urethan und Allophanate. Dieser Strom wird in den Spaltreaktor zurückgeleitet. Der Reaktoraustragsstrom 2 von ca. 1,3 kg/h enthält ca. 70 - 80 Gew.-% höher siedende Verbindungen wie z.B. Allophanate.

Die Ausbeute an Hexamethylendiisocyanat, bezogen auf das eingesetzte Urethan, beträgt ca. 67%.

### Beispiel 2

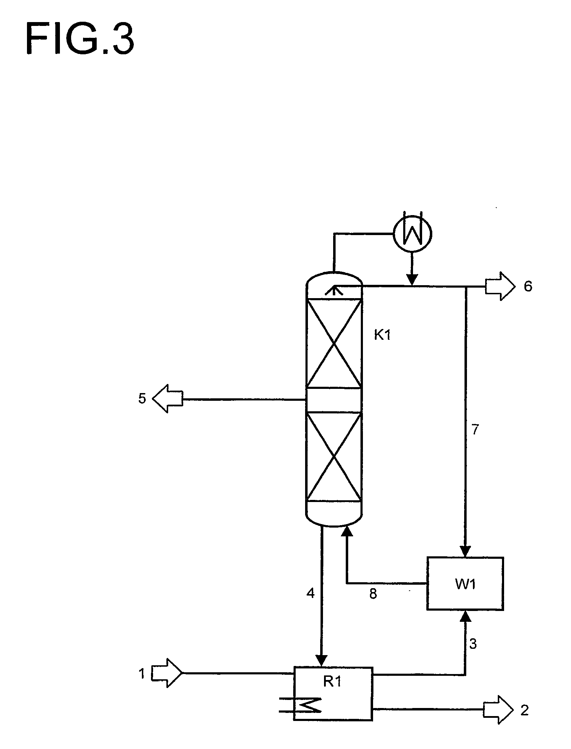

Die Spaltreaktion und Aufarbeitung der Spaltgase wird in der in Figur 3 dargestellten Vorrichtung durchgeführt. Diese gleicht der Vorrichtung gemäß Figur 1 (Vergleichsbeispiel 2), jedoch werden die Gase, die den Spaltreaktor verlassen, in einem separaten Kühler W1 mit n-Butanol abgekühlt und der Rektifizierkolonne zugeführt. Die Rektifizierkolonne konnte durch die Verwendung des separaten Kühlers W1 auf 10 theoretische Trennstufen reduziert werden. Die Reaktionsbedingungen im Spaltreaktor sind mit denen im Vergleichsbeispiel 2 identisch. In den Spaltreaktor R1 wird ein Reaktorzulauf 1 von 4 kg/h aus 89 Gew.-% Hexamethylendiurethan dosiert. Zur Abkühlung des Spaltgasstroms 3 von 8,1 kg/h mit einem Anteil von ca. 20 Gew.-% Hexamethylendiisocyanat wird von dem im Kopf der Kolonne K1 anfallenden Strom 6 aus flüssigem n-Butanol ein Strom 7 von ca. 0,5 kg/h n-Butanol in den separaten Kondensator W1 gepumpt. Zusätzlich wird am Kondensator W1 mittels einer Temperierung noch ein Wärmestrom von 0,2 kW abgeführt. Der diesen Kondensator verlassende Stoffstrom 8 mit einem Gasanteil von ca. 80% und einer Temperatur von ca. 200°C wird in die Rektifizierkolonne K1 eingeleitet. Aus dem Sumpf der Kolonne K1 wird ein Strom 4 von ca, 5,2 kg/h mit etwa 78 Gew.-% halbgespaltenem Urethan und einer Temperatur von ca. 180°C abgezogen. Im Seitenabzug wird ein Strom 5 der Kolonne K1 von 1,4 kg/h Hexamethylendiisocyanat mit einer Konzentration von 97,5 Gew.-% erhalten. Am Kopf der Kolonne K1 fällt ein Strom 6 von ca. 1,4 kg/h flüssigem n-Butanol mit einer Reinheit von 97 Gew.-% an.

Die Ausbeute an Hexamethylendiisocyanat verbesserte sich dadurch von 67 auf 72%.

### Beispiel 3

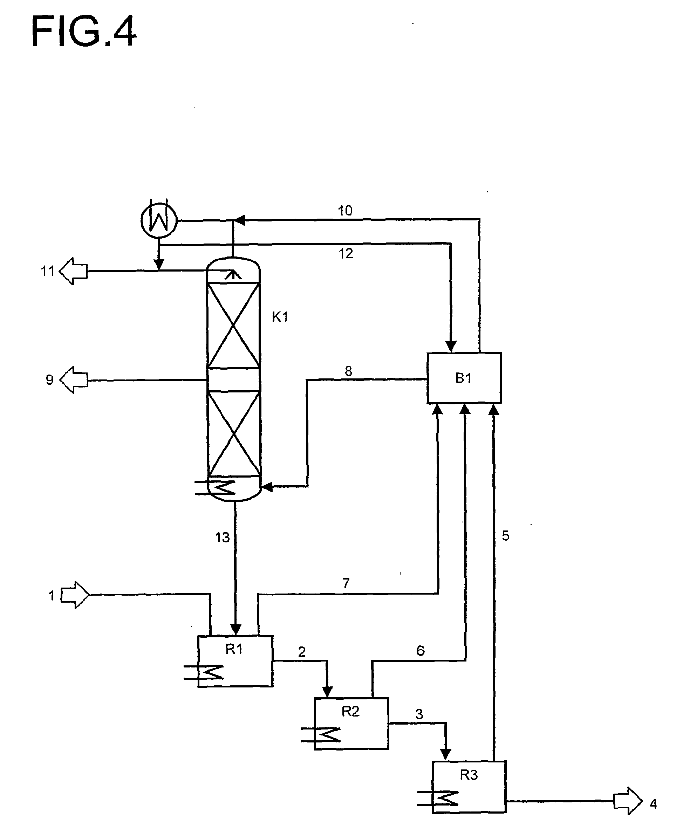

Die Spaltreaktion und Aufarbeitung der Spaltgase wird in der in Figur 4 dargestellten Vorrichtung durchgeführt. Die Spaltreaktion erfolgt in einer Kaskade aus Reaktoren R1, R2 und R3. Die mittlere Verweilzeit des Reaktionsgemischs in den Reaktoren liegt zwischen 2 und 10 Minuten, die Temperatur beträgt zwischen 238 und 244 °C und der Druck beträgt ca. 160 mbar. In den ersten Spaltreaktor R1 werden 4 kg/h eines Zulaufs 1, enthaltend ca. 92 Gew.-% Hexamethylendi-n-butylurethan, eingespeist. Dem letzten Reaktor R3 wird ein Flüssigkeitsstrom 4 von ca. 0,5 kg/h entnommen. Die den Spaltreaktoren entnommenen Spaltgasströme 5, 6 und 7 werden in den Kondensator B1, umfassend Kühlschlangen und einen Kühlmantel, eingespeist. Dem Kondensator B1 wird ein Strom 12 von 0,9 kg/h n-Butanol zugeführt und ein Kondensatstrom 8 von 5,5 kg/h entnommen. Der Kondensatstrom 8 enthält ca. 43 Gew.-% n-Butanol, 36 Gew.-% Hexamethylendiisocyanat, 2 Gew.-% Hexamethylendi-n-butylurethan und 16 Gew.-% halbgespaltenes Urethan. Der Kondensatstrom 8 wird auf der Höhe der ersten theoretischen Stufe in die Rektifizierkolonne K1 eingespeist. Die Kolonne besitzt 20 theoretische Trennstufen und ist mit einer Metallpackung der Fa. MONZ gefüllt. Die Sumpftemperatur in der Kolonne liegt bei ca. 190 °C, der Kopfdruck beträgt ca. 20 mbar. Auf Höhe der 5. theoretischen Stufe werden 1,8 kg/h Hexamethylendiisocyanat mit einer Reinheit von 98 Gew.-%, entsprechend einer Ausbeute von > 90 %, als Seitenabzug 9 entnommen. Als Sumpfabzug wird ein Strom 13 von 1,2 kg/h, enthaltend 50 Gew.-% Hexamethylendiisocyanat und Allophanate, entnommen und in den Reaktor R1 zurückgeführt. Am Kolonnenkopf wird ein Strom 11 von 1,6 kg/h n-Butanol mit einer Reinheit von 98 Gew.-% entnommen. Der den Kondensator B 1 verlassende dampfförmige Strom 10 von ca. 0,1 kg/h enthält zu 99 Gew.-% n-Butanol und wird direkt im Kopfkondensator der Rektifizierkolonne K1 eingespeist.

## Patentansprüche

1. Verfahren zur Herstellung von Isocyanaten aus Carbamidsäureestern, bei dem man
(i) ein Gemisch enthaltend einen einfach oder mehrfach funktionellen Carbamidsäureester kontinuierlich in einen Spaltreaktor oder eine Kaskade aus zwei oder mehr Spaltreaktoren einleitet,
(ii) den Carbamidsäureester einer thermischen Spaltung zu Isocyanat und Alkohol unterwirft,
(iii) das dem oder den Spaltreaktoren entweichende Spaltgas, enthaltend das Isocyanat, den Alkohol, gegebenenfalls ungespaltenen und/oder teilweise gespaltenen Carbamidsäureester sowie Nebenkomponenten, zumindest teilweise mit einem flüssigen Kühl- und Kondensationsmittel in Kontakt bringt, abkühlt und zumindest teilweise kondensiert,
(iv) aus dem abgekühlten Spaltgas und dem Kondensat in einer Rektifizierkolonne das Isocyanat und den Alkohol gewinnt,
wobei ein Teilstrom des gewonnenen Alkohols als flüssiges Kühl- und Kondensationsmittel zur Abkühlung und Kondensation des Spaltgases eingesetzt wird.

2. Verfahren nach Anspruch 1, **dadurch gekennzeichnet, dass** man den Kühl- und Kondensationsschritt (iii) in der Rektifizierkolonne durchführt, indem man das Spaltgas am Kolonnensumpf in die Rektifizierkolonne einspeist, den Teilstrom des gewonnenen Alkohols im unteren Kolonnenabschnitt, vorzugsweise auf Höhe des 1. bis 4. theoretischen Bodens, in die Rektifizierkolonne einspeist und dem aufsteigenden Spaltgas entgegenführt.

3. Verfahren nach Anspruch 1, **dadurch gekennzeichnet, dass** man den Kühl- und Kondensationsschritt (iii) in einem separaten Kondensator durchführt.

4. Verfahren nach einem der Ansprüche 1 bis 3, **dadurch gekennzeichnet, dass** man den Alkohol als Kopfabzugsstrom gewinnt, und das Isocyanat als Seitenabzugsstrom gewinnt.

5. Verfahren nach einem der Ansprüche 1 bis 4, **dadurch gekennzeichnet, dass** man den Sumpfabzugsstrom der Rektifizierkolonne, enthaltend ungespaltenen und gegebenenfalls teilweise gespaltenen Carbamidsäureester, in den Spaltreaktor bzw. die Spaltreaktorkaskade zurückführt.

6. Verfahren nach einem der Ansprüche 1 bis 5, **dadurch gekennzeichnet, dass** der oder die Spaltreaktoren Robertverdampfer, Herbertverdampfer, Caddletype-Verdampfer, Rohrbündelreakrtoren oder Kessel mit innenliegender Heizschlange sind.

7. Verfahren nach einem der Ansprüche 1 bis 6, **dadurch gekennzeichnet, dass** die thermische Spaltung des Carbamidsäureesters in Abwesenheit eines zusätzlichen Lösungsmittels durchgeführt wird.

8. Verfahren nach einem der Ansprüche 1 bis 7, **dadurch gekennzeichnet, dass** der Carbamidsäureester Hexamethylendi-n-butylurethan, das Isocyanat Hexamethylendiisocyat und der Alkohol n-Butanol sind.

## Claims

1. A process for the preparation of isocyanates from carbamic esters, in which
(i) a mixture comprising a monofunctional or polyfunctional carbamic ester is passed continuously into a cleavage reactor or a cascade of two or more cleavage reactors,
(ii) the carbamic ester is subjected to a thermal cleavage to give isocyanate and alcohol,
(iii) the cleavage gas escaping from the cleavage reactor or reactors and comprising the isocyanate, the alcohol, any uncleaved and/or partly cleaved carbamic ester and byproducts is at least partly brought into contact with a liquid coolant and condensing agent, cooled and at least partly condensed and
(iv) the isocyanate and the alcohol are obtained from the cooled cleavage gas and the condensate in a rectification column,
a part-stream of the alcohol obtained being used as liquid coolant and condensing agent for cooling and condensing the cleavage gas.

2. The process according to claim 1, wherein the cooling and condensation step (iii) is carried out in the rectification column by feeding the cleavage gas into the rectification column at the bottom of the column, feeding the part-stream of the alcohol obtained into the rectification column in the lower column section, preferably at the height of the 1st to 4th theoretical plate, and passing said part-stream countercurrent to the ascending cleavage gas.

3. The process according to claim 1, wherein the cooling and condensation step (iii) is carried out in a separate condenser.

4. The process according to any of claims 1 to 3, wherein the alcohol is obtained as a top take-off stream and the isocyanate is obtained as a side take-off stream.

5. The process according to any of claims 1 to 4, wherein the bottom take-off stream of the rectification column, comprising uncleaved and any partly cleaved carbamic ester, is recycled into the cleavage reactor or the cleavage reactor cascade.

6. The process according to any of claims 1 to 5, wherein the cleavage reactor or reactors is or are a Robert evaporator, Herbert evaporator, Caddletype evaporator, tube-bundle reactor or kettle having an internal heating coil.

7. The process according to any of claims 1 to 6, wherein the thermal cleavage of the carbamic ester is carried out in the absence of an additional solvent.

8. The process according to any of claims 1 to 7, wherein the carbamic ester is hexamethylenedi-n-butylurethane, the isocyanate is hexamethylene diisocyanate and the alcohol is n-butanol.

## Revendications

1. Procédé de fabrication d'isocyanates à partir d'esters de l'acide carbamique, selon lequel
(i) un mélange contenant un ester de l'acide carbamique mono- ou polyfonctionnel est introduit en continu dans un réacteur de clivage ou une cascade de deux réacteurs de clivage ou plus,
(ii) l'ester de l'acide carbamique est soumis à un clivage thermique en isocyanate et alcool,
(iii) le gaz de clivage sortant du ou des réacteurs de clivage, qui contient l'isocyanate, l'alcool, éventuellement de l'ester de l'acide carbamique non clivé et/ou partiellement clivé, ainsi que des composants secondaires, est mis en contact au moins partiellement avec un agent de refroidissement et de condensation liquide, refroidi et au moins partiellement condensé,
(iv) l'isocyanate et l'alcool sont extraits à partir du gaz de clivage refroidi et du condensat dans une colonne de rectification,
un courant partiel de l'alcool extrait étant utilisé en tant qu'agent de refroidissement et de condensation liquide pour refroidir et condenser le gaz de clivage.

2. Procédé selon la revendication 1, **caractérisé en ce que** l'étape de refroidissement et de condensation (iii) est réalisée dans la colonne de rectification, en introduisant le gaz de clivage dans la colonne de rectification au fond de la colonne, en introduisant le courant partiel de l'alcool extrait dans la colonne de rectification dans la section inférieure de la colonne, de préférence à hauteur du 1^{e} au 4^{e} plateau théorique, et en le mettant en circulation en sens inverse du gaz de clivage ascendant.

3. Procédé selon la revendication 1, **caractérisé en ce que** l'étape de refroidissement et de condensation (iii) est réalisée dans un condensateur distinct.

4. Procédé selon l'une quelconque des revendications 1 à 3, **caractérisé en ce que** l'alcool est extrait en tant que courant de sortie de tête et l'isocyanate est extrait en tant que courant de sortie latérale.

5. Procédé selon l'une quelconque des revendications 1 à 4, **caractérisé en ce que** le courant de sortie de fond de la colonne de rectification, qui contient de l'ester de l'acide carbamique non clivé et éventuellement partiellement clivé, est recyclé dans le réacteur de clivage ou la cascade de réacteurs de clivage.

6. Procédé selon l'une quelconque des revendications 1 à 5, **caractérisé en ce que** le ou les réacteurs de clivage sont des évaporateurs Robert, des évaporateurs Herbert, des évaporateurs de type Caddle, des réacteurs à faisceau de tubes ou des cuves à serpentin chauffant interne.

7. Procédé selon l'une quelconque des revendications 1 à 6, **caractérisé en ce que** le clivage thermique de l'ester de l'acide carbamique est réalisé en l'absence d'un solvant supplémentaire.

8. Procédé selon l'une quelconque des revendications 1 à 7, **caractérisé en ce que** l'ester de l'acide carbamique est l'uréthane di-n-butylique d'hexaméthylène, l'isocyanate est le diisocyanate d'hexaméthylène et l'alcool est le n-butanol.
